Europäisches Patentamt

European Patent Office

Office européen des brevets

(19)

(11) Veröffentlichungsnummer: **0 355 506 B1**

(12) **EUROPÄISCHE PATENTSCHRIFT**

(45) Veröffentlichungstag der Patentschrift: **14.12.94**

(51) Int. Cl.5: **A61B 5/0476**

(21) Anmeldenummer: **89114280.4**

(22) Anmeldetag: **02.08.89**

(54) **Anordnung zum Messen lokaler bioelektrischer Ströme in biologischen Gewebekomplexen.**

(30) Priorität: **16.08.88 DE 3827799**

(43) Veröffentlichungstag der Anmeldung:
**28.02.90 Patentblatt 90/09**

(45) Bekanntmachung des Hinweises auf die
Patenterteilung:
**14.12.94 Patentblatt 94/50**

(84) Benannte Vertragsstaaten:
**DE FR IT NL**

(56) Entgegenhaltungen:
**US-A- 4 532 591**
**US-A- 4 736 751**
**US-A- 4 753 246**

**MEDICAL AND BIOLOGICAL ENGINEERING
AND COMPUTING. vol. 15, no. 6, November
1977, STEVENHAGE GB Seiten 641 - 647; S.M.
Mason et al.: "Simple online detector of auditory evoked cortical potentials"**

(73) Patentinhaber: **SIEMENS AKTIENGESELL-
SCHAFT
Wittelsbacherplatz 2
D-80333 München (DE)**

(72) Erfinder: **Abraham-Fuchs, Klaus, Dipl.-Phys.
Graslitzer Strasse 17
D-8520 Erlangen (DE)**
Erfinder: **Schneider, Siegfried, Dr.
Kulmbacher Strasse 33
D-8520 Erlangen (DE)**
Erfinder: **Röhrlein, Gerhard, Dr.
Biengarten 25
D-8552 Höchstadt (DE)**

EP 0 355 506 B1

**Beschreibung**

Die Erfindung betrifft eine Anordnung zum Messen lokaler bioelektrischer Ströme in biologischen Gewebekomplexen nach dem Oberbegriff des Patentanspruchs 1. Eine solche Anordnung ist aus der US-PS 4,736,751 bekannt. Sie dient dazu, Hirnaktivitäten auf statistischer Basis mit Hilfe eines Digitalrechners zu analysieren. Dazu werden in der üblichen Weise Sensoren über der Schädeldecke verteilt, deren Meßsignale digitalisiert und gespeichert werden. Der zu untersuchende Patient wird dabei einem Satz von stimulierenden Sinnesreizen ausgesetzt, die in lokalisierbaren Hirnbereichen elektrische oder magnetische Felder auslösen. Diese Felder werden von den Sensoren in elektrische Signale umgesetzt, die statistisch ausgewertet und analysiert werden, um den jeweiligen Ort der Aktivität zu verifizieren. Aufgrund der räumlichen und zeitlichen Zusammenhänge dieser spontanen Ereignisse kann der Ort ihrer Entstehung und ihrer Ausbreitung an einem dreidimensionalen Schädelmodell, hergestellt etwa durch ein Computertomogramm nach der magnetischen Resonanzmethode (MR), dargestellt werden.

Der Nachteil dieser Methode besteht darin, daß sie nur dann zu brauchbaren Ergebnissen führt, wenn die evozierten Potentiale groß genug sind, um sich aus dem Rauschpegel deutlich erkennbar herauszuheben. Dies trifft bei evozierten Potentialen im allgemeinen nach einer größeren Anzahl von Reizen und deren Mittelwertbildung zu. Die Methode eignet sich aber nicht ohne weiteres zur Feststellung sogenannter spontaner Ereignisse, wie sie etwa bei einem epileptischen Anfall auftreten. Diese machen sich nach dem heutigen Stand des Wissens im Elektroenzephalogramm (EEG) durch charakteristische Signalmuster, z.B. sogenannten "Spike and Wave-Komplexen" von etwa 200 bis 500 ms Dauer bemerkbar. Diese Signalmuster sind auch zwischen akuten Anfällen (interiktal) feststellbare, allerdings mit von Patient zu Patient sehr unterschiedlicher Häufigkeit. Solche Signalmuster können in Extremfällen jede Sekunde oder auch nur einmal pro Woche auftreten. Aufgrund des geringen Signal-Rausch-Verhältnisses sind solche interiktalen Signalmuster im EEG meist nur schwer und nur von erfahrenen Neurologen, im Magnetoenzephalogramm (MEG) mit bloßem Auge praktisch gar nicht erkennbar. Der Ursprungsort solcher spontan auftretenden Signalmuster wird als epileptogener Herd gedeutet. Ziel der Auswertung eines EEG oder MEG in der Epilepsie-Diagnostik ist es, den Ort dieses Herdes möglichst exakt zu lokalisieren. Außerdem ist für den Neurologen von Bedeutung, Aussagen über die räumliche Ausbreitung signalbildender elektrischer Erregungen sowohl innerhalb eines Signalmusters als auch bei aufeinanderfolgenden unterschiedlichen Signalmustern zu gewinnen. Solche Aussagen sind bisher nur invasiv mittels EEG-Tiefenelektroden und auch damit nur sehr eingeschränkt möglich. Eine zeitauflösende Lokalisierung war bisher praktisch nicht zu erreichen, da aufgrund des geringen Signal-Rausch-Verhältnisses aus einem einzelnen Signalereignis eine Lokalisierung mit der erforderlichen Genauigkeit nicht möglich ist und für eine Mittelung von Signalereignissen in der Regel nicht genügend solche Ereignisse zur Verfügung standen.

Der Erfindung liegt die Aufgabe zugrunde, die von über dem zu untersuchenden biologischen Gewebekomplex räumlich verteilt angeordneten Sensoren für die Messung von elektrischen oder magnetischen Feldstärkewerten ausgehenden elektrischen Signale derart zu verarbeiten, daß bestimmte, innerhalb eines Zeitintervalls auftretende Signalmuster als Grundlage für das Aufsuchen ähnlicher Muster aus dem laufenden Signal dienen können sowie deren zeitliches Auftreten und deren räumliche Zuordnung festzustellen.

Diese Aufgabe wird durch die im Patentanspruch 1 und 2 angegebene Erfindung gelöst. Die darin vorgeschlagene räumliche und zeitliche Korrelation der gemittelten Signalmuster zeigt brauchbare Signalwerte, die sich ausreichend vom Rauschen abheben und entsprechend ausgewertet werden können.

In einer Weiterbildung der Erfindung gemäß Patentanspruch 3 ist erreicht, daß auswertbare Störfrequenzen, wie beispielsweise die Netzfrequenz oder bestimmte periodisch auftretende spontane Biosignale aus den Signalkanälen der Sensoren ausgefiltert werden können.

Gemäß einer weiteren Ausbildung nach Patentanspruch 4 wird die räumliche Korrelation durch die Mittelung der Korrelationsfunktion über das zugeordnete Zeitintervall des Signalmusters und die zeitliche Korrelationsfunktion mit anschließender Mittelwertbildung über den zugeordneten Raum nach bestimmten mathematischen Zusammenhängen gebildet. Durch deren anschließende Multiplikation können Spitzensignale gewonnen werden, die sich besonders deutlich aus dem Rauschen abheben.

Die in Patentanspruch 2 beschriebene Anordnung ermöglicht die Bestimmung der für einen Meßdatensatz charakteristischen Ähnlichkeitsschwelle.

Die erforderlichen Rechenoperationen nehmen, wenn sie von den üblichen Digitalrechnern ausgeführt werden, eine erhebliche Rechenzeit in Anspruch. Bei einer Weiterbildung der Erfindung nach Patentanspruch 7 ist durch die Verwendung eines Arrayprozessor-Rechners in Zusammenhang mit einem Algorithmus zur sogenannten schnellen Faltung eine wesentliche Verkürzung der Rechenzeit erzielbar, die es erlaubt, Ergebnisse kurz nach Ablauf der Untersuchung zu erhalten, so daß der Patient für eine eventuelle Wiederholung mit der Meßanordnung verbunden bleiben kann.

Zwar ist es aus "Biogmagnetism" 1987, "Proceedings of the 6th International Conference on Biomagnetism" unter dem Titel "New Method for the Study of Spontaneous Brain Aktivity" zur Detektion von epileptischen und von Alpha-Aktivitäten bekannt, eine Korrelation der Ortsmuster zur Erkennung von "Spike-Wave-Komplexen" in nur einem EEG-Kanal vorzunehmen. Diese Methode ist jedoch nur dann ausreichend, wenn ein signifikantes Ereignis in einem Kanal deutlich erkennbar ist. Es hat sich jedoch gezeigt, daß im Rahmen der in der Praxis auftretenden Signal-Rausch-Verhältnisse signifikante Korrelationen von "Spike-Wave-Komplexen" bei der einfachen Korrelationsfunktion gar nicht und in den räumlich und zeitlich gemittelten Korrelationsfunktionen eines MEG nur sehr schlecht zu beobachten sind. Erst durch die Behandlung der Signalkomplexe nach der vorliegenden Erfindung heben sich die charakteristischen Signalmuster so deutlich vom Rauschen ab, daß sie für die weitere Signalbehandlung und Signalauswertung verwendet werden können.

Weitere Einzelheiten der Erfindung ergeben sich aus den nachfolgenden Erläuterungen zu einem in den Figuren 1 bis 4 dargestellten Ausführungsbeispiel. Darin zeigen:

FIG 1    eine Meßanordnung für die Behandlung der von den am Patienten angeordneten Sensoren gelieferten Meßwerte;

FIG 2    ein typisches Signalmuster eines spontanen Ereignisses im Hirnbereich eines Patienten;

FIG 3    das nach einer Raum-zeitlichen Korrelation erhaltene gemittelte Signal gemäß FIG 2;

FIG 4    eine typische Verteilungskurve, die die Häufigkeit des Auftretens eines bestimmten Ähnlichkeitsmaßes (Korrelationskoeffizienten) zwischen der Signalaufzeichnung und dem Vergleichssignal darstellt;

FIG 5    eine Schaltungsanordnung zur Bestimmung der Ähnlichkeitsschwelle; und

FIG 6    das von einem Computertomographen gewonnene Schichtbild des Gehirns mit einer eingezeichneten typischen Erregungsbahn eines spontanen Ereignisses.

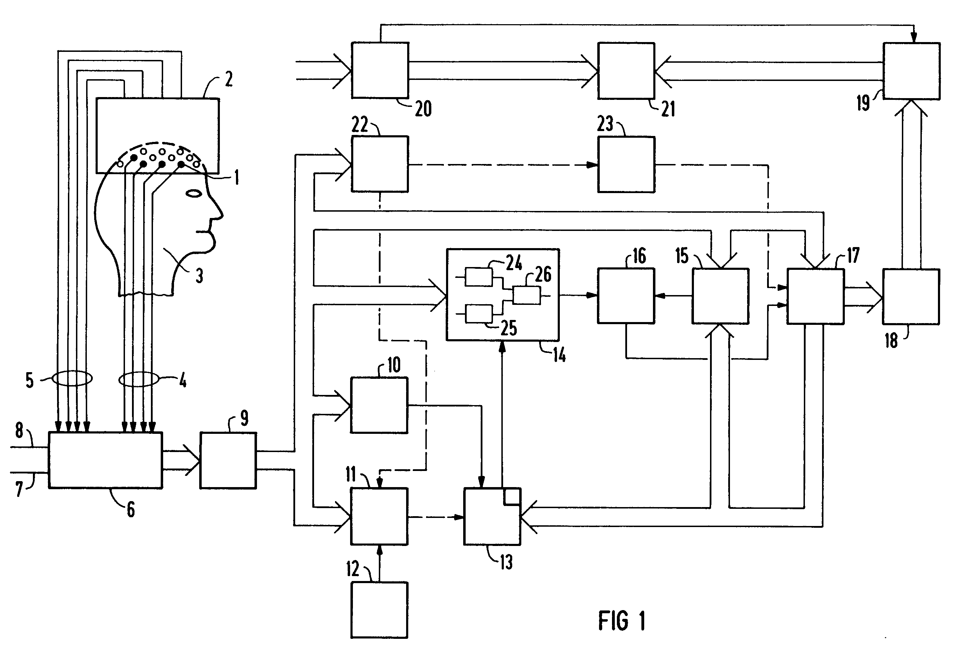

In FIG 1 sind die Sensorelektroden 1 eines Elektroenzephalographen (EEG) sowie der Squid-Sensor eines Mehrkanal-Magnetoenzephalographen (MEG) 2 über der Schädelkalotte 3 eines Patienten räumlich verteilt angeordnet. Die Sensoren erzeugen den gemessenen elektrischen bzw. magnetischen Feldern entsprechende elektrische Signale, die über Zuleitungen 4, 5 einem N-Kanal A/D-Wandler 6 zugeführt sind. Weiterhin können dem A/D-Wandler Triggersignale aus einem EKG-Gerät über eine Zuleitung 7 und von der Atmung gesteuerte Triggersignale über die Zuleitung 8 zugeführt werden, die in bekannter Weise dazu dienen, die Meßwerterfassung innerhalb bestimmter, von Atemfrequenz und/oder Herztätigkeit bestimmter zeitlicher Grenzen auszulösen. Die Digitalsignale der EEG- und MEG-Kanäle werden einem N-kanaligen digitalen Frequenzfilter 9 zugeleitet, der periodisch auftretende Störfrequenzen, wie beispielsweise die Netzfrequenz oder Erregungszentren der vom Hirn ausgehenden, sogenannten Alpha-Wellen, ausfiltert. Der Ausgang des Frequenzfilters 9 ist mit einem EEG-Monitor 10 verbunden, der die Ausgangssignale in auswertbarer Form (digital oder analog) anzeigt und damit einer Analyse durch den Arzt zugänglich macht.

Alternativ besteht die Möglichkeit, anstelle des EEG-Monitors 10 eine frei programmierbare Mustererkennungsstufe 11 vorzusehen, die mittels eines Programmiergerätes 12 für die Erkennung bestimmter Signalmuster programmierbar ist.

Das erkannte Signalmuster muß dann zeitlich definiert werden. Dazu werden ein Anfangszeitpunkt und ein Endzeitpunkt festgelegt. Ein solches zeitlich definiertes Signalmuster wird als "Template" bezeichnet. Das so erkannte und definierte Template wird in der Template-Speicherstufe 13 abgespeichert.

Das fortlaufend gemessene Signal am Ausgang des digitalen Frequenzfilters 9 wird einer Korrelationsstufe 14 zugeführt, die auch das in der Template-Speicherstufe 13 gespeicherte Template abrufen kann, um es mit dem laufend eingehenden Signal zu vergleichen. Dazu wird das als Template definierte Zeitintervall als "Zeitfenster" über die eingehenden Daten geschoben. In jedem Zeitfenster wird der Korrelationskoeffizient jedes zeitlich korrespondierenden Signalmusters mit Hilfe seines ersten Rechenschaltkreises 24 nach der folgenden mathematischen Beziehung berechnet und über alle Meßorte gemittelt:

$$K_R(t_i) = \frac{1}{N} \sum_{C_i=C_1}^{C_N} \left( \frac{\sum_{\tau_i=\tau_0}^{\tau_M} s_{C_i}(t_i+\tau_i)\, s_{C_i}(\tau_i)}{\sqrt{\sum_{\tau_i=\tau_0}^{\tau_M} s_{C_i}^2(t_i+\tau_i)} \cdot \sqrt{\sum_{\tau_i=\tau_0}^{\tau_M} s_{C_i}^2(\tau_i)}} \right)$$

Ebenso wird der Korrelationskoeffizient der Signalkurven in dem betreffenden Fenster mit Hilfe eines zweiten Rechenschaltkreises 25 nach der folgenden Formel

$$K_T(t_i) = \frac{1}{M} \sum_{\tau_i=\tau_0}^{\tau_M} \left( \frac{\sum_{c_i=c_1}^{c_N} s_{c_i}(t_i + \tau_i) \cdot s_{c_i}(\tau_i)}{\sqrt{\sum_{c_i=c_1}^{c_N} s_{c_i}^2(t_i + \tau_i)} \cdot \sqrt{\sum_{c_i=c_1}^{c_N} s_{c_i}^2(\tau_i)}} \right)$$

an jedem Meßort berechnet und über alle Zeitpunkte des Fensters gemittelt. Die so berechneten Zeitfunktionen der zeitlichen und räumlichen Korrelation werden anschließend mit Hilfe eines dritten Rechenschaltkreises 26 nach der Formel

$$K_{RT}(t_i) = K_R(t_i) \cdot K_T(t_i)$$

multipliziert.

Darin bedeuten:

$C_1 \ldots C_N$ N magnetische Meßkanäle in beliebiger Ortsverteilung

$$s_{c_i}(t_i)$$

magnetisches Signal im Kanal $C_i$ zum Zeitpunkt $t_i$

$\tau_0 \ldots \tau_i \ldots \tau_M$ Zeitintervall des Templates, Beginn $\tau_o$, Ende $\tau_M$, mit M Abtastwerten im Zeitintervall;

$\tau_i$ Zeitpunkt im Zeitintervall $\tau_o \leqq \tau_i \leqq \tau_M$

$K_T(t_i)$ zeitlicher Korrelationsfaktor, (Korrelationskoeffizient zum Zeitpunkt $t_i$ von Template und Meßsignal)

$K_R(t_i)$ räumlicher Korrelationsfaktor (Korrelationskoeffizient zum Zeitpunkt $t_i$ von Template und Meßsignal)

$K_{RT}(t_i)$ Raum-zeitlicher Korrelationsfaktor

Das so erhaltene, nach der korrelierten Raum-Zeit-Funktion gebildete Korrelationssignal wird einer Vergleichsstufe 16 zugeführt, welche dieses Korrelationssignal mit einem Schwellwertsignal vergleicht, das in einer Schwellwertdefinitionsstufe 15 aus dem Ausgangssignal des Frequenzfilters 9 gewonnen wird. Bei Überschreiten der Schwelle wird das Schwellwertsignal einer Mittelungsstufe 17 zugeführt. Diese bildet ein Mittelwertsignal aller aus den vorgenannten Kriterien erkannter Signalmuster über der Zeit an allen Meßorten, welches einerseits der Template-Speicherstufe 13 und andererseits der Schwellendefinitionsstufe 15 zur laufenden Korrektur der Templates zugeführt ist. Darüber hinaus werden die von der Mittelungsstufe 17 gemittelten Signalmuster einer Lokalisierungsstufe 18 zugeführt, welche den geometrischen Ort der aufgetretenen pathologischen elektrisch aktiven Quelle berechnet und diese Daten einer Koordinatentransformationsstufe 19 zuführt, die das Koordinatensystem der EEG-bzw. MEG-Messung mit demjenigen, beispielsweise eines in der Bildspeicherstufe 20 gespeicherten Computertomogramms, zur Deckung bringt, so daß beide Darstellungen auf einem Monitor 21 als Schnittbild oder als räumliches Bild zur Darstellung gebracht werden können.

Eine alternative, von Fall zu Fall zu besseren Ergebnissen führende Methode zur Auffindung sehr schwacher Signalmuster besteht darin, daß mit Hilfe einer Betragssummenstufe 22 die Betragssummen der Signale aller Kanäle gebildet und einer Mustererkennungsstufe 23 zugeführt werden, die der Mustererkennungsstufe 11, 12 entspricht. Das erkannte Muster wird anstelle des von der Vergleichsstufe 16 kommenden Signals der Mittelungsstufe 17 zugeführt und in der beschriebenen Weise bearbeitet. Das Summensignal kann alternativ auch der Mustererkennungsstufe 11, 12 zugeführt und zur Templatedefinition verwendet werden. Diese Art der Signalverarbeitung eignet sich besonders bei einer Signalerzeugung nur über die MEG-Abtastung.

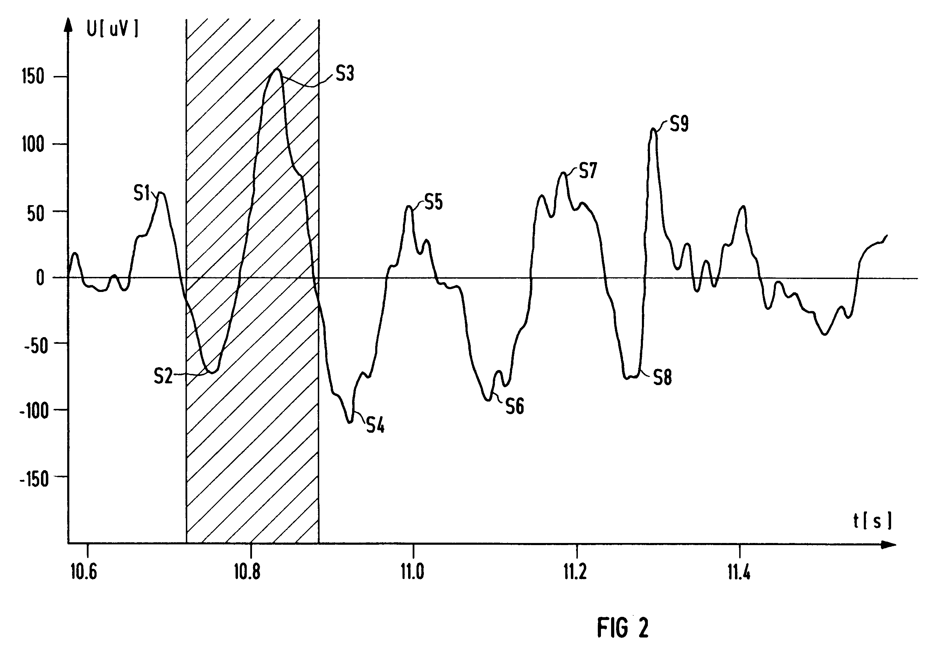

In FIG 2 ist ein EEG-Kurvenzug des von einer EEG-Elektrode erzeugten Signalverlaufs dargestellt, bei dem mit bloßem Auge dreieckförmige Signale S1 bis S9 auffallen, die vom Neurologen als "Sharp-Wave" bezeichnet werden, deren pathologische Signifikanz allerdings nicht eindeutig ist. Dabei hebt sich der

4

Komplex $S_2$, $S_3$ von den übrigen ab. Dieser wurde daher als Template verwendet und ist schraffiert dargestellt.

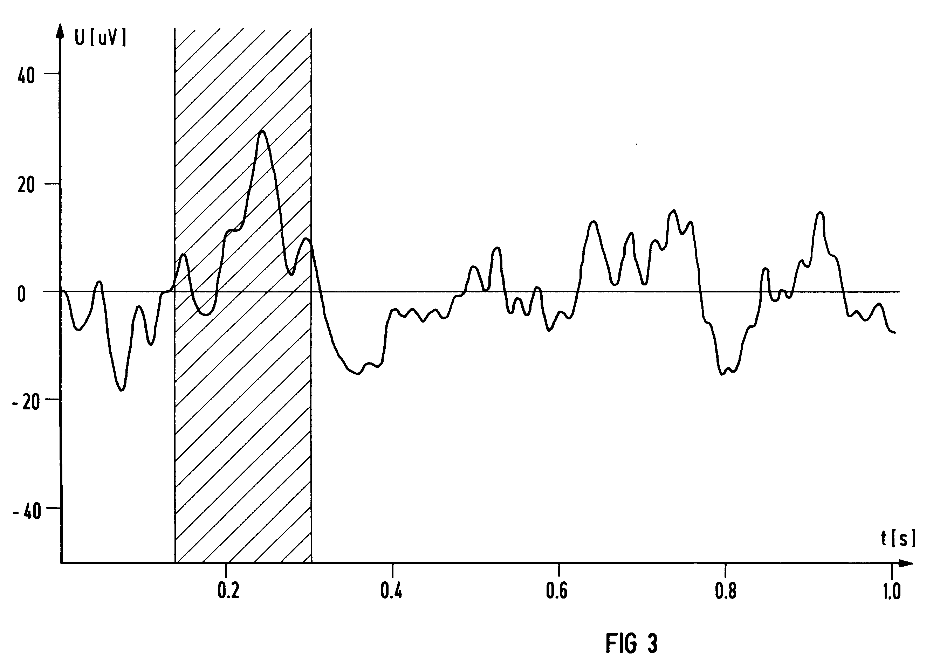

In FIG 3 ist das nach der raum-zeitlichen Korrelation erhaltene gemittelte Signal im selben EEG-Kanal schraffiert dargestellt. Das so gemittelte Signalmuster erfüllt erheblich deutlicher die Kriterien eines pathologischen "Spike-Wave-Komplexes", zeigt jedoch eine kompliziertere Struktur, als sie sich in dem bisher bekannten EEG darstellte.

Die bisher beschriebene Anordnung erlaubt das Erkennen bestimmter Ereignisse aus einer kontinuierlichen Aufzeichnung bioelektrischer oder biomagnetischer Signale mittels einer digitalen raum-zeitlichen Korrelationsanalyse durch den Vergleich der kontinuierlich eintreffenden Signalaufzeichnung mit einem gespeicherten definierten Signalmuster (template).

Als Ergebnis dieses Vergleichs erhält man zu jedem Vergleichszeitpunkt im Datensatz für den Korrelationskoeffizienten einen Wert zwischen -1 und +1, der ein Maß ist für die Ähnlichkeit der Signalaufzeichnung innerhalb des vom Signalmuster bestimmten Zeitfensters in jedem Vergleichszeitpunkt. Ist der Korrelationskoeffizient gleich +1, so ist die Übereinstimmung unter gleichem Vorzeichen maximal. Der Korrelationskoeffizient erreicht bei Null die schlechteste Übereinstimmung und bei -1 eine maximale Übereinstimmung, unter umgekehrtem Vorzeichen der Signale. Die weitere Ausbildung der Erfindung hat zum Ziel, nicht nur jene Signalbereiche aus der Signalfolge herauszufinden, die mit dem Template identisch sind, sondern auch ein charakteristisches Maß an Ähnlichkeit aufweisen. Es sollen also diejenigen Signalbereiche registriert werden, die eine für den betreffenden Datensatz charakteristische Ähnlichkeitsschwelle überschreiten.

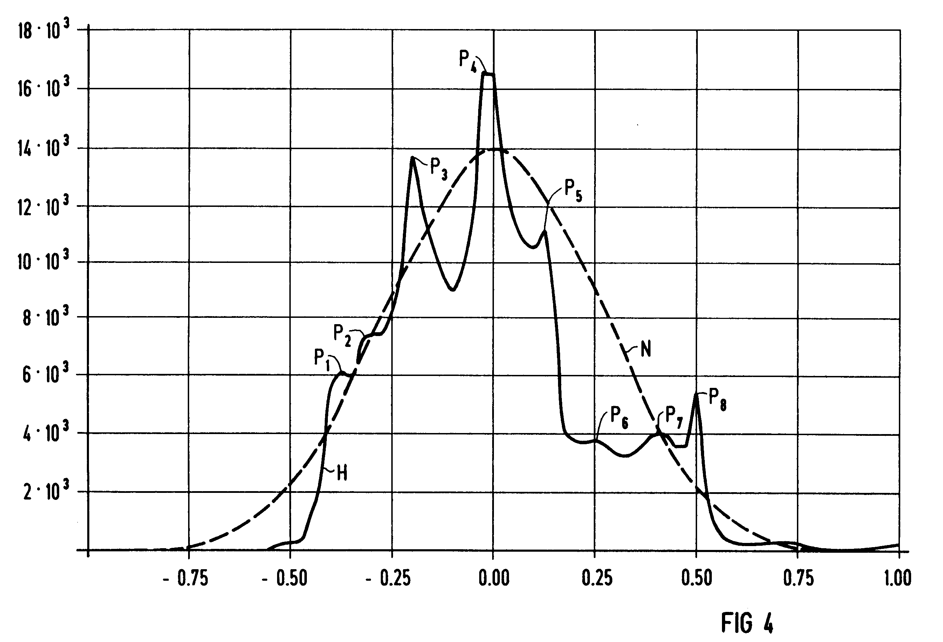

In Fig. 4 ist anhand einer typischen Verteilungskurve die Häufigkeit des Auftretens aller möglichen Ähnlichkeitsmaße zwischen der Signalaufzeichnung und dem Vergleichssignal dargestellt. Wenn der untersuchte Signalbereich nur aus weißem Rauschen besteht, stellt die Häufigkeitsverteilung aller Korrelationskoeffizienten eine Gaus'sche Normalverteilung dar, wie sie als gestrichelte Kurve N in Fig. 4 gezeigt ist. Jede Abweichung der Häufigkeitsverteilung, dem sogenannten Histogramm, von der Normalverteilung, wie sie etwa in der ausgezogenen Kurve H in Fig. 4 dargestellt ist, ist ein eindeutiges Zeichen dafür, daß Signalkomplexe vorhanden sind, die, je nach der Größe ihres jeweiligen Korrelationskoeffizienten, eine mehr oder weniger große Ähnlichkeit zum vorgegebenen Kurvenverlauf des Templates besitzen. Solche Abweichungen äußern sich in Spitzen $P_1$ ... $P_8$, die die Normalverteilungskurve N überlagern. Je näher eine solche Spitze am Wert +1 liegt, umso größer ist das Ähnlichkeitsmaß. Demzufolge bestimmt der links (d.h. in Richtung kleiner Korrelationskoeffizienten) liegende Fußpunkt derjenigen Spitze, die am nähesten am Korrelationskoeffizienten +1 liegt, die gesuchte charakteristische Ähnlichkeitsschwelle. Im vorliegenden Fall würde also die gesuchte Ähnlichkeitsschwelle aus der Spitze $P_8$ mit 0,48 bestimmt werden. Jede Überschreitung dieses Schwellwertes definiert einen Zeitpunkt im untersuchten Signal, der eine ausreichende Ähnlichkeit mit dem Template besitzt.

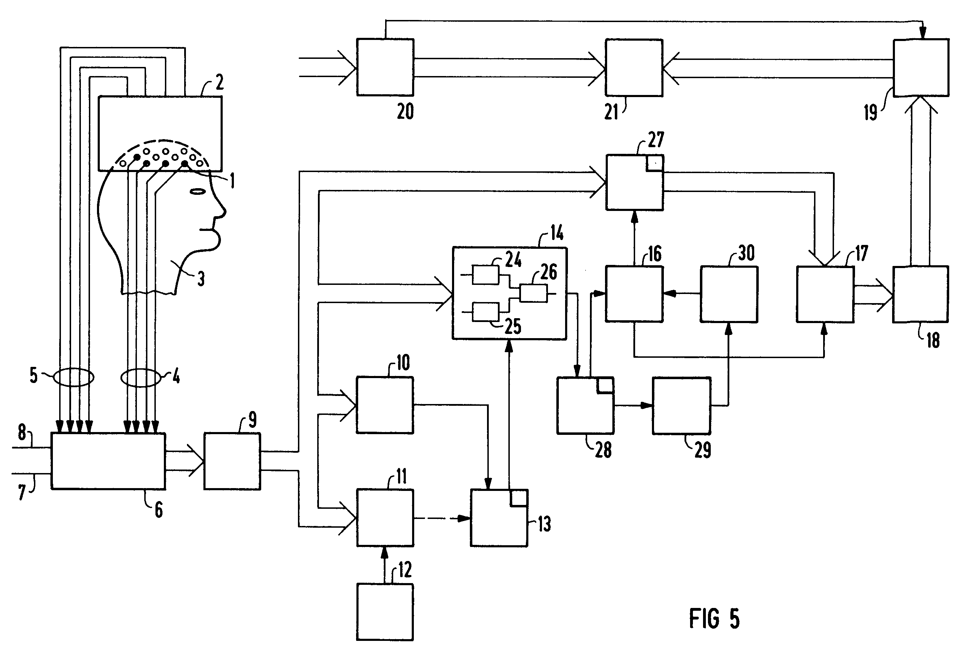

Eine Schaltungsanordnung zur Bestimmung der Ähnlichkeitsschwelle ist in Fig. 5 dargestellt. Dabei sind diejenigen Stufen, die mit denjenigen in Fig. 1 funktionsgleich sind, mit gleichen Bezugszeichen versehen.

Der Unterschied zu der in Fig. 1 gezeigten Schaltungsanordnung besteht darin, daß das Meßsignal in der Speicherstufe 27 und das von der Korrelationsstufe 14 gebildete Korrelationssignal in einer Speicherstufe 28 gespeichert wird. Dieses Signal wird einer Rechenstufe 29 zur Berechnung des Histogramms und gleichzeitig der Vergleichsstufe 16 zugeführt. Das am Ausgang der Rechenstufe 29 auftretende Histogramm-Signal wird einer Schwellwertbestimmungsstufe 30 zugeleitet, die aus der Verteilungskurve den charakteristischen Schwellwert bestimmt und ebenfalls der Vergleichsstufe 16 zuführt. In der Vergleichsstufe 16 wird nun das gespeicherte Korrelationssignal aus der Speicherstufe 28 mit dem charakteristischen Schwellwert aus der Schwellwertbestimmungsstufe 30 verglichen und und bei Überschreitung des Schwellwertes der zu diesem Zeitpunkt gehörende Signalabschnitt der Mittelungsstufe 17 zugeführt und von dort über die Lokalisierungsstufe 18 wie bereits beschrieben ausgewertet. Auf diese Weise werden auch solche Signale für die Auswertung erfaßt, die eine charakteristische Ähnlichkeitsschwelle zum Template-Signal überschreiten, so daß auch bei nicht bekannter Rausch-Amplitude oder bei einer Summierung des Gesamtsignals aus gesuchtem Signal, Rauschen und anderen charakteristischen Signalkomplexen eine Erkennung der gesuchten Signalkomplexe mit nachfolgender Mittelwertbildung möglich ist.

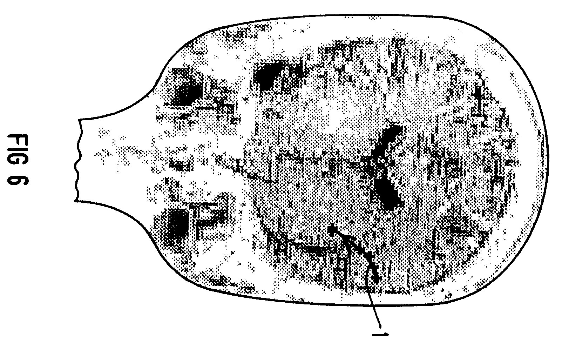

In FIG 6 ist das auf dem Monitor 20 erkennbare Bild dargestellt, bei dem das Computertomogramm mit dem koordinatentransformierten MEG-Lokalisationsbild zur Deckung gebracht ist. Daraus ist der Bereich der pathologischen elektrischen Aktivität durch die mit Kreuzen markierten Punkte durch eine den zeitlichen Aktivitätsverlauf charakterisierende Linie deutlich erkennbar.

**Patentansprüche**

1. Anordnung zum Lokalisieren bioelektrischer Stromquellen in biologischen Gewebekomplexen mit über diesen räumlich verteilt angeordneten Sensoren zur Bildung elektrischer Meßwerte aufgrund von gemessenen, von den bioelektrischen Stromquellen erzeugten elektrischen oder magnetischen Feldgrößen mit
   - einer Stufe (17) für eine Mittelwertbildung von gleichartigen Signalmustern,
   - mit einer Stufe (18) zur räumlichen Zuordnung der gemittelten Signalmuster zu den signalauslösenden Stromquellen und
   - mit einer Stufe (21) für die räumliche Darstellung der Stromquellen innerhalb einer Abbildung des Gewebekomplexes, **gekennzeichnet** durch eine Signalverarbeitungsanordnung, bestehend aus einer Stufe (11,12,13) für eine visuelle oder automatische Erkennung einzelner, in den Meßwerten vorhandener Signalmuster, eine zeitliche Begrenzung des Signalmusters (Zeitfenster) und eine Abspeicherung der in dem Zeitfenster liegenden Meßwerte von jedem Sensor (template), einer weiteren Stufe (14) für eine räumliche und zeitliche Korrelation der fortlaufenden Meßwerte jedes Sensors mit dem innerhalb des Zeitfenster liegenden, dem jeweiligen Sensor zugeordneten Signalmuster zur Bildung eines Korrelationssignals, einer Schwellwertdefinitionsstufe (15), die aus den fortlaufenden Meßwerten einen Schwellwert erzeugt, einer Vergleichsstufe (16), die das Korrelationssignal mit dem Schwellwert vergleicht und die bei Überschreiten des Schwellwertes fortlaufende Meßwerte, aus denen das schwellwertüberschreitende Korrelationssignal gebildet wurde, der Stufe (17) für Mittelwertbildung zuführt.

2. Anordnung zum Lokalisieren bioelektrischer Stromquellen in biologischen Gewebekomplexen mit über diesen räumlich verteilt angeordneten Sensoren zur Bildung elektrischer Meßwerte aufgrund von gemessenen, von den bioelektrischen Stromquellen erzeugten elektrischen oder magnetischen Feldgrößen mit
   - einer Stufe (17) für eine Mittelwertbildung von gleichartigen Signalmustern,
   - mit einer Stufe (18) zur räumlichen Zuordnung der gemittelten Signalmuster zu den signalauslösenden Stromquellen und
   - mit einer Stufe (21) für die räumliche Darstellung der Stromquellen innerhalb einer Abbildung des Gewebekomplexes,
   **gekennzeichnet** durch eine Signalverarbeitungsanordnung, bestehend aus einer Stufe (11,12,13) für eine visuelle oder automatische Erkennung einzelner, in den Meßwerten vorhandener Signalmuster, eine zeitliche Begrenzung des Signalmusters (Zeitfenster) und eine Abspeicherung der in dem Zeitfenster liegenden Meßwerte von jedem Sensor (template), einer weiteren Stufe (14) für eine räumliche und zeitliche Korrelation der fortlaufenden Meßwerte jedes Sensors mit dem innerhalb des Zeitfensters liegenden, dem jeweiligen Sensor zugeordneten Signalmuster zur Bildung eines Korrelationssignals, einer Speicherstufe (28) zur Zwischenspeicherung des Korrelationssignals und einer Rechenstufe (19) zur Berechnung einer Häufigkeitsverteilung (Histogramm) des gespeicherten Korrelationssignals, einer Schwellwertbestimmungsstufe (30), die aufgrund einer definierten Abweichung der Häufigkeitsverteilung von einer Gaußschen Normalverteilungskurve eine für den untersuchten Signalabschnitt charakteristische Ähnlichkeitsschwelle ermittelt und einer Vergleichsstufe (16) zuleitet, die das zwischengespeicherte Korrelationssignal mit dem Ähnlichkeitsschwellwert vergleicht und bei Überschreitung fortlaufende Meßwerte, aus denen das schwellwertüberschreitende Korrelationssignal gebildet wurde, aus der Speicherstufe (27) der Mittelungsstufe (17) zuführt.

3. Anordnung nach Anspruch 1 oder 2, **dadurch gekennzeichnet,** daß die von den Sensoren (1, 2) gebildeten elektrischen Signalmuster durch einen den Sensoren (1,2) nachgeordneten Analog-Digital-Wandler (6) digitalisiert und einem digitalen Frequenzfilter (9) zugeführt werden, welcher auswählbare Störfrequenzen aus den Signalkanälen (4,5) ausfiltert.

4. Anordnung nach einem der Ansprüche 1 bis 3, **dadurch gekennzeichnet,** daß die Stufe (14) für die räumliche und zeitliche Korrelation gebildet ist aus einem ersten Rechenschaltkreis (24) für die Bildung der zeitlichen Korrelationsfunktion und deren Mittelung über den zugeordneten Raum nach der Formel

$$K_R(t_i) = \frac{1}{N} \sum_{C_i=C_1}^{C_N} \frac{\sum_{\tau_i=\tau_0}^{\tau_M} S_{C_i}(t_i + \tau_i)\, S_{C_i}(\tau_i)}{\sqrt{\sum_{\tau_i=\tau_0}^{\tau_M} S_{C_i}^2(t_i + \tau_i)} \cdot \sqrt{\sum_{\tau_i=\tau_0}^{\tau_M} S_{C_i}^2(\tau_i)}}$$

einem zweiten Schaltkreis (25) zur Bildung der räumlichen Korrelationsfunktion mit anschließender Mittelung über das zugeordnete Zeitfenster nach der Formel

$$K_T(t_i) = \frac{1}{M} \sum_{\tau_i=\tau_0}^{\tau_M} \frac{\sum_{C_i=C_1}^{C_N} S_{C_i}(t_i + \tau_i) \cdot S_{C_i}(\tau_i)}{\sqrt{\sum_{C_i=C_1}^{C_N} S_{C_i}^2(t_i + \tau_i)} \cdot \sqrt{\sum_{C_i=C_1}^{C_N} S_{C_i}^2(\tau_i)}}$$

und einen dritten Rechenschaltkreis (26) für die Multiplikation der beiden Funktionen, wobei die Formelzeichen bedeuten:

$C_1 \ldots C_N$  N magnetische Meßkanäle in beliebiger Ortsverteilung

$$S_{C_i}(t_i)$$

magnetisches Signal im Kanal $C_i$ zum Zeitpunkt $t_i$

$\tau_0 \ldots \tau_i \ldots \tau_M$ Zeitintervall des Templates, Beginn $\tau_0$, Ende $\tau_M$, mit M Abtastwerten im Zeitintervall;

$\tau_i$ Zeitpunkt im Zeitintervall $\tau_0 \leq \tau_i \leq \tau_M$

$K_T(t_i)$ zeitlicher Korrelationsfaktor, (Korrelationskoeffizient zum Zeitpunkt $t_i$ von Template und Meßsignal)

$K_R(t_i)$ räumlicher Korrelationsfaktor (Korrelationskoeffizient zum Zeitpunkt $t_i$ von Template und Meßsignal)

$K_{RT}(t_i)$ Raum-zeitlicher Korrelationsfaktor

5.  Anordnung nach Anspruch 1 oder 2, **dadurch gekennzeichnet,** daß die Stufe (14) für die räumliche und zeitliche Korrelation einen einzigen Rechenschaltkreis enthält, der die Korrelation nach der Formel

$$K_{RT}(t_i) = \frac{\sum_{C_i=C_1}^{C_N} \sum_{\tau_i=\tau_0}^{\tau_M} S_{C_i}(t_i + \tau_i) \cdot S_{C_i}(\tau_i)}{\sqrt{\sum_{C_i=C_1}^{C_N} \sum_{\tau_i=\tau_0}^{\tau_M} S_{C_i}^2(t_i + \tau_i)} \cdot \sqrt{\sum_{C_i=C_1}^{C_N} \sum_{\tau_i=\tau_0}^{\tau_M} S_{C_i}^2(\tau_i)}}$$

vornimmt, wobei die Formelzeichen die in Anspruch 4 definierte Bedeutung haben.

6. Anordnung nach einem der Ansprüche 3 bis 5 in Verbindung mit Anspruch 1, **dadurch gekennzeichnet,** daß das von der Mittelwertstufe (17) gebildete Mittelwertsignal der Templatespeicherstufe (13) und/oder der Schwellwertdefinitionsstufe (15) zur laufenden Korrektur der Templates zugeführt ist.

7. Anordnung nach einem der vorhergehenden Ansprüche, **dadurch gekennzeichnet,** daß für die Bildung der räumlich-zeitlichen Korrelationsfunktionen ein Arrayprozessor-Rechner benutzt ist, und daß die Korrelationsfunktion unter Ausnutzung eines Algorithmus zur "schnellen Faltung" gebildet wird.

**Claims**

1. An arrangement for localizing bioelectrical current sources in biological tissue complexes with sensors arranged spatially distributed over said tissue complexes for the formation of electrical measured values on the basis of measured electrical or magnetic field quantities produced by the bioelectrical current sources with
   - a stage (17) for the averaging of similar signal patterns,
   - with a stage (18) for the spatial assignment of the averaged signal patterns to the signal-triggering current sources and
   - with a stage (21) for the spatial representation of the current sources within an image of the tissue complex, characterised by a signal processing arrangement consisting of a stage (11, 12, 13) for visual or automatic recognition of individual signal patterns present in the measured values, temporal limitation of the signal pattern (time window), and storage of the measured values, present in the time window, from each sensor (template), a further stage (14) for spatial and temporal correlation of the continuous measured values of each sensor with the signal pattern present within the time window and assigned to the respective sensor for the formation of a correlation signal, a threshold value definition stage (15) which produces a threshold value from the continuous measured values, a comparator stage (16) which compares the correlation signal with the threshold value and on the overshooting of the threshold value supplies continuous measured values, from which the correlation signal overshooting the threshold value has been formed, to the averaging stage (17).

2. An arrangement for the localization of bioelectrical current sources in biological tissue complexes with sensors arranged spatially distributed over said tissue complexes for the formation of electrical measured values on the basis of measured electrical or magnetic field quantities produced by the bioelectrical current sources with
   - a stage (17) for the averaging of similar signal patterns,
   - with a stage (18) for the spatial assignment of the averaged signal patterns to the signal-triggering current sources and
   - with a stage (21) for the spatial representation of the current sources within an image of the tissue complex, characterised by a signal processing arrangement consisting of a stage (11, 12, 13) for visual or automatic recognition of individual signal patterns present in the measured values, temporal limitation of the signal pattern (time window), and storage of the measured values present in the time window from each sensor (template), a further stage (14) for spatial and temporal correlation of the continuous measured values of each sensor with the signal pattern which is present within the time window and is assigned to the respective sensor for the formation of a correlation signal, a storage stage (28) for the intermediate storage of the correlation signal and with a calculating stage (19) for the calculation of a frequency distribution (histogram) of the stored correlation signal, a threshold value determining stage (30) which, on the basis of a defined deviation of the frequency distribution from a Gaussian normal distribution curve, establishes a similarity threshold characteristic of the investigated signal portion and supplies the latter to a comparator stage (16) which compares the intermediately stored correlation signal with the similarity threshold value and in the event of the overshooting of the threshold value supplies continuous measured values, from which the correlation signal overshooting the threshold value has been formed, from the storage stage (27) to the averaging stage (17).

3. An arrangement as claimed in Claim 1 or 2, characterised in that the electrical signal patterns formed by the sensors (1, 2) are digitalized by an analogue-digital converter (6) arranged at the output end of the sensors (1, 2) and are fed to a digital frequency filter (9) which filters out selectable interference frequencies from the signal channels (4, 5).

4. An arrangement as claimed in one of Claims 1 to 3, characterised in that the stage (14) for the spatial and temporal correlation is formed from a first calculating circuit (24) for the formation of the temporal correlation function and its averaging over the assigned space in accordance with the formula

$$K_R(t_i) = \frac{1}{N} \sum_{C_i=C_1}^{C_N} \frac{\sum_{\tau_i=\tau_0}^{\tau_M} s_{C_i}(t_i + \tau_i)\, s_{C_i}(\tau_i)}{\sqrt{\sum_{\tau_i=\tau_0}^{\tau_M} s_{C_i}^2(t_i + \tau_i)} \cdot \sqrt{\sum_{\tau_i=\tau_0}^{\tau_M} s_{C_i}^2(\tau_i)}}$$

a second circuit (25) for the formation of the spatial correlation function with subsequent averaging over the assigned time window in accordance with the formula

$$K_T(t_i) = \frac{1}{M} \sum_{\tau_i=\tau_0}^{\tau_M} \frac{\sum_{C_i=C_1}^{C_N} s_{C_i}(t_i + \tau_i) \cdot s_{C_i}(\tau_i)}{\sqrt{\sum_{C_i=C_1}^{C_N} s_{C_i}^2(t_i + \tau_i)} \cdot \sqrt{\sum_{C_i=C_1}^{C_N} s_{C_i}^2(\tau_i)}}$$

and a third calculating circuit (26) for the multiplication of the two functions, where the formula symbols have the following significance:

$C_1 \dots C_N$ N magnetic measuring channels in arbitrary local distribution

$$s_{C_i}(t_i)$$

magnetic signal in the channel $C_i$ at the time $t_i$

$\tau_0 \dots \tau_i \dots \tau_M$ time interval of the template, start $\tau_0$, end $\tau_M$, with M sample values in the time interval; $\tau_i$ time in the time interval $\tau_0 \leq \tau_i \leq \tau_M$

$K_T(t_i)$ temporal correlation factor, (correlation coefficient at the time $t_i$ of template and measured signal)

$K_R(t_i)$ spatial correlation factor (correlation coefficient at the time $t_i$ of template and measured signal)

$K_{RT}(t_i)$ space-time correlation factor.

5. An arrangement as claimed in Claim 1 or 2, characterised in that the stage (14) for the spatial and temporal correlation comprises one single calculating circuit which undertakes the correlation in accordance with the formula

$$K_{RT}(t_i) = \frac{\sum_{C_i=C_1}^{C_N} \sum_{\tau_i=\tau_0}^{\tau_M} s_{C_i}(t_i + \tau_i) \cdot s_{C_i}(\tau_i)}{\sqrt{\sum_{C_i=C_1}^{C_N} \sum_{\tau_i=\tau_0}^{\tau_M} s_{C_i}^2(t_i + \tau_i)} \cdot \sqrt{\sum_{C_i=C_1}^{C_N} \sum_{\tau_i=\tau_0}^{\tau_M} s_{C_i}^2(\tau_i)}}$$

where the formula symbols have the significance defined in Claim 4.

6. An arrangement as claimed in one of Claims 3 to 5 in association with Claim 1, characterised in that the average signal formed by the averaging stage (17) is fed to the template storage stage (13) and/or to the threshold value definition stage (15) for the continuous correction of the templates.

7. An arrangement as claimed in one of the preceding claims, characterised in that an array processor computer is used for the formation of the space-time correlation functions, and that the correlation function is formed using a "fast convolution" algorithm.

**Revendications**

1. Dispositif pour localiser des sources de courant bioélectriques dans des complexes de tissus biologiques, comportant des capteurs disposés en étant répartis dans l'espace au-dessus de ces complexes et servant à former des valeurs électriques de mesure sur la base de grandeurs de champs électriques ou magnétiques mesurées et produites par les sources de courant bioélectriques, comportant
   - un étage (17) pour la formation de la valeur moyenne de modèles de signaux de même type,
   - un étage (18) pour réaliser l'association spatiale des modèles de signaux, dont la moyenne est formée, aux sources de courant déclenchant des signaux, et
   - un étage (21) pour la représentation spatiale des sources de courant à l'intérieur d'une image du complexe de tissu,
   caractérisé par un dispositif de traitement des signaux, qui est constitué par un étage (11,12,13) pour une identification visuelle ou automatique de différents modèles de signaux présents dans les valeurs de mesure, une limitation latérale du modèle de signal (fenêtre temporelle) et une mémorisation des valeurs de mesure, situées dans la fenêtre temporelle et fournies par chaque capteur (template), un autre étage (14) pour une corrélation spatiale et temporelle des valeurs de mesure suivantes délivrées en continu par chaque capteur, avec le modèle de signal associé à chaque capteur et situé à l'intérieur de la fenêtre temporelle, pour la formation d'un signal de corrélation, un étage de définition de valeur de seuil (15), qui produit une valeur de seuil à partir des valeurs de mesure arrivant continûment, un étage comparateur (16), qui compare le signal de corrélation à la valeur de seuil et qui, lors du dépassement de la valeur de seuil, envoie les valeurs de mesure délivrées continûment et à partir desquelles a été formé le signal de corrélation qui dépasse la valeur de seuil, à l'étage (17) de formation de la valeur moyenne.

2. Dispositif pour localiser des sources de courant bioélectriques dans des complexes de tissus biologiques, comportant des capteurs disposés en étant répartis dans l'espace au-dessus de ces complexes et servant à former des valeurs électriques de mesure sur la base de grandeurs de champs électriques ou magnétiques mesurées et produites par les sources de courant bioélectriques, comportant
   - un étage (17) pour la formation de la valeur moyenne de modèles de signaux de même type,
   - un étage (18) pour réaliser l'association spatiale des modèles de signaux, dont la moyenne est formée, aux sources de courant déclenchant des signaux, et
   - un étage (21) pour la représentation spatiale des sources de courant à l'intérieur d'une image du complexe de tissu,
   caractérisé par un dispositif de traitement des signaux, qui est constitué par un étage (11,12,13) pour une identification visuelle ou automatique de différents modèles de signaux présents dans les valeurs de mesure, une limitation latérale du modèle de signal (fenêtre temporelle) et une mémorisation des valeurs de mesure, situées dans la fenêtre temporelle et fournies par chaque capteur (template), un autre étage (14) pour une corrélation spatiale et temporelle des valeurs de mesure suivantes délivrées en continu par chaque capteur, avec le modèle de signal associé à chaque capteur et situé à l'intérieur de la fenêtre temporelle, pour la formation d'un signal de corrélation, un étage de définition de valeur de seuil (15), un étage de mémoire (28) pour mémoriser temporairement le signal de corrélation et un étage de calcul (19) pour calculer une distribution de fréquences (histogramme) du signal de corrélation mémorisé, un étage (30) de détermination d'une valeur de seuil, qui, sur la base d'un écart défini de la distribution de fréquences par rapport à une courbe de distribution gaussienne normale, détermine un seuil de similitude caractéristique de la partie de signal étudiée, et l'envoie à un étage comparateur (16), qui compare le signal de corrélation mémorisé temporairement à la valeur de seuil de similitude et, lors du dépassement de cette valeur, envoie des valeurs de mesure délivrées continûment, à partir desquelles le signal de corrélation dépassant la valeur de seuil a été formé, depuis l'étage de mémoire (27) à l'étage (17) de formation de la moyenne.

3. Dispositif suivant la revendication 1 ou 2, caractérisé par le fait que le modèle de signal électrique, formé par les capteurs (1,2), est numérisé par un convertisseur analogique/numérique (6), disposé en aval des capteurs (1,2), et sont envoyés à un filtre numérique de fréquence (9), qui sépare, par filtrage des fréquences parasites pouvant être sélectionnées, des canaux de transmission de signaux (4,5).

4. Dispositif suivant l'une des revendications 1 à 3, caractérisé par le fait que l'étage (14) pour la corrélation spatiale et temporelle est formé par un premier circuit de calcul (24) servant à former la fonction de corrélation temporelle et sa moyenne dans l'espace associé, conformément à la formule

$$K_R(t_i) = \frac{1}{N} \sum_{C_i=C_1}^{C_N} \frac{\sum_{\tau_i=\tau_0}^{\tau_M} S_{C_i}(t_i+\tau_i) S_{C_i}(\tau_i)}{\sqrt{\sum_{\tau_i=\tau_0}^{\tau_M} S_{C_i}^2(t_i+\tau_i)} \cdot \sqrt{\sum_{\tau_i=\tau_0}^{\tau_M} S_{C_i}^2(\tau_i)}}$$

par un second circuit pour la formation de la fonction de corrélation temporelle et avec formation ultérieure de la moyenne dans la fenêtre temporelle associée, conformément à la formule

$$K_T(t_i) = \frac{1}{M} \sum_{\tau_i=\tau_0}^{\tau_M} \frac{\sum_{C_i=C_1}^{C_N} S_{C_i}(t_i+\tau_i) \cdot S_{C_i}(\tau_i)}{\sqrt{\sum_{C_i=C_1}^{C_N} S_{C_i}^2(t_i+\tau_i)} \cdot \sqrt{\sum_{C_i=C_1}^{C_N} S_{C_i}^2(\tau_i)}}$$

et par un troisième circuit de calcul (26) pour la multiplication de ces deux fonctions, les symboles dans les formules désignant ce qui suit :
$c_1 ... C_N$ N canaux magnétiques de mesure selon une répartition locale quelconque,
$S_{Ci}(t_i)$ signal magnétique dans le canal $C_i$ à l'instant $t_i$,
$t_0...t_i...t_M$ intervalle de temps du gabarit, début $t_0$, fin $t_M$, avec M valeurs d'échantillonnage pendant l'intervalle de temps; $t_i$ instants dans l'intervalle $t_0 \leq t_i \leq t_M$,
$K_T(t_i)$ facteur de corrélation temporelle (coefficient de corrélation à l'instant $t_i$ pour le gabarit de signal de mesure),
$K_R(t_i)$ facteur de corrélation spatiale (coefficient de corrélation à l'instant $t_i$ entre le gabarit et le signal de mesure,
$K_{RT}(t_i)$ facteur de corrèlation spatio-temporelle.

5. Dispositif suivant la revendication 1 ou 2, caractérisé par le fait que l'étage (14) pour les corrélations spatiale et temporelle contient un seul circuit de calcul, qui exécute la corrélation conformément à la formule

EP 0 355 506 B1

$$K_{RT}(t_i) = \frac{\displaystyle\sum_{c_i=c_1}^{\dot{c}_N} \sum_{\tau_i=\tau_0}^{\tau_M} {}^{S}c_i \, (t_i + \tau_i) \cdot {}^{S}c_i \, (\tau_i)}{\sqrt{\displaystyle\sum_{c_i=c_1}^{c_N} \sum_{\tau_i=\tau_0}^{\tau_M} {}^{S}c_i^{\,2} \, (t_i + \tau_i)} \cdot \sqrt{\displaystyle\sum_{c_i=c_1}^{c_N} \sum_{\tau_i=\tau_0}^{\tau_M} {}^{S}c_i^{\,2} \, (\tau_i)}}$$

les symboles de la formule ayant les significations définies dans la revendication 4.

6. Dispositif suivant l'une des revendications 3 à 5 en liaison avec la revendication 1, caractérisé par le fait que le signal de valeur moyenne, formé par l'étage (17) de formation de la valeur moyenne, est envoyé à l'étage de mémoire de gabarits (13) et/ou à l'étage (15) de définition de valeurs de seuil, pour une correction permanente du gabarit.

7. Dispositif suivant l'une des revendications précédentes, caractérisé par le fait que pour la formation des fonctions de corrélation spatio-temporelle, on utilise un calculateur à processeur vectoriel, et que la fonction de corrélation est formée moyennant l'utilisation d'un algorithme de "convolution rapide".

12

FIG 1

EP 0 355 506 B1

FIG 2

EP 0 355 506 B1

FIG 3

EP 0 355 506 B1

FIG 4

EP 0 355 506 B1

FIG 5

FIG 6